# EUROPEAN PATENT APPLICATION

(11) **EP 1 746 413 A2**
(43) Date of publication of application: **24.01.2007**
(21) Application number: 06253472.2
(22) Date of filing: 01.07.2006
(51) Int. Cl.: G01N 27/327, C12Q 1/00, G01N 33/487

(54) **Electrochemical Biosensor and method of manufacture**

(30) Priority: 19.07.2005 GB 0514728
(71) Applicant: HYPOGUARD LIMITED, Woodbridge Suffolk IP12 1PE (GB)
(72) Inventor: Rippeth, John J., Ipswich, IP4 1NL (GB); Ho, Wah On, Colchester, CO4 5WS (GB)
(74) Representative: Gemmell, Peter Alan

(57) **Abstract**

An electrochemical biosensor (12) includes a working electrode (5) and a counter electrode (3), wherein at least a part of the working electrode (5) comprises an enzyme and is disposed between and in electrical connection with two separate conductive tracks (1,2). Other aspects of the invention provide a meter (26) for use with the biosensor (12) and a method of determining whether a fluid sample applied to the biosensor (12) has sufficiently wetted the working electrode (5).

## Description

### BACKGROUND OF THE INVENTION

### 1. Field of the Invention

The present invention relates to a biosensor for measuring analyte concentration in a biological fluid, notably in whole blood. The invention also provides a method of manufacturing the biosensor. Biosensors typically include a working electrode comprising an enzyme layered on or mixed with an electrically conductive substrate. The electrodes respond electrochemically to the catalytic activity of the enzyme in the presence of a suitable analyte. Other aspects of the invention provide a meter for use with the biosensor and a method for determining whether a fluid sample has sufficiently wetted a working electrode of the biosensor.

### 2. Description of the Prior Art

Electrochemical biosensors are well known in the art. They are used in measurement techniques including amperometry, coulometry and potentiometry. Typically the enzyme is an oxidoreductase, for example glucose oxidase, cholesterol oxidase, or lactate oxidase, which produces hydrogen peroxide according to the reaction:

analyte+O₂-[oxidase]→oxidised product+H₂O₂.

In an amperometric measurement, the peroxide is oxidised at a fixed-potential electrode as follows:

H₂O₂→O₂+2H⁺+2e⁻.

Electrochemical oxidation of hydrogen peroxide at platinum centres on the electrode results in transfer of electrons from the peroxide to the electrode producing a current which is proportional to the analyte concentration. Where glucose is the analyte, the oxidised product is gluconolactone.

In coulometric measurement, the total current passed during completion or near completion of electrolysis of the analyte is measured and integrated to give a value of charge passed. The charge passed is related to the quantity of analyte present in a sample so that if the sample volume is known the analyte concentration can be determined. In potentiometric measurement, a potential generated by the reaction is measured at one or more points in time and related to the initial analyte concentration. The various electrochemical measurement techniques are well known to those skilled in the art.

Typically, electrochemical measurement begins automatically when the blood sample completes an electrical circuit between the working and counter electrodes. Getting an accurate reading can be a problem when a blood sample incompletely covers the working electrode because the amount of current or measured charge is less than when the working electrode is fully covered. If a user attempts to top-up the sample by applying a second drop of blood ('double-dosing') this has the effect of reducing the precision of the measurement and increasing the response as the addition of extra blood causes a non-faradaic charging peak to occur when more of the electrode area is covered by the second sample.

It has been proposed to reduce the problem of incomplete fill by employing a pair of fill-detection electrodes in the fluid path, with the working and counter electrodes inbetween. A measurement is only taken when a circuit has been completed between the fill electrodes. However, this arrangement adds complexity to the system and does not address the problems of double-dosing by the user.

### SUMMARY OF THE INVENTION

According to an aspect of the present invention there is provided a biosensor which includes a working electrode and a counter electrode, wherein at least a part of the working electrode is disposed between and in electrical connection with two separate conductive tracks.

Other aspects of the invention provide a test meter as specified in claim 13, a method of determining whether a fluid sample has sufficiently wetted the working electrode of the biosensor as specified in claim 18, and a method of manufacturing the biosensor as specified in claim 30. Preferred features are specified in the dependent claims.

By connecting the working electrode between two separate conductive tracks, the biosensor may be used to detect electrically when the working electrode has become wetted with a biological fluid, for example whole blood. Other suitable biological fluids include plasma, serum, tears, sweat, and urine. A preferred detection method is to measure the electrical resistance between the conductive tracks. The resistance will change when the working electrode is substantially wetted. In an alternative method, a change in impedance is indicative of the working electrode being wetted. The impedance may increase or decrease depending on how the measurement is performed.

The invention is of particular utility where the biosensor is a capillary-fill biosensor, in which the working electrode and the counter electrode are located in a capillary flow path which extends inwardly from an edge of the biosensor. Such biosensors require only small (sub-µL) sample volumes. The use of small sample volumes is desirable to enable sampling to be taken from alternative sites on a user's body, such as the forearm, upper arm, or thigh instead of from a user's finger. There are lower densities of pain receptors in alternative sites, but those sites tend to produce less blood when lanced.

In a preferred embodiment, the electrodes are arranged such that a fluid sample which flows evenly along the capillary flow path from the edge will substantially completely cover the working electrode before the fluid sample makes contact with any part of the counter electrode. This arrangement enables detection of incomplete fill, where the sample does not bridge the working and counter electrodes within a pre-set time after it wets the working electrode. The user may be prompted to discard the biosensor and try again. Alternatively, this arrangement also allows double-dosing without loss of precision or increased values resulting from an extra non-faradaic charging peak. Because the first dose does not complete the circuit between the working and counter electrode only a single non-faradaic charging peak occurs, after sufficient extra sample fluid has been added in a second or subsequent dose.

In a preferred embodiment, the biosensor is used with a test meter which can detect 'creep fill', where a small finger of sample connects the working electrode and the counter electrode. Creep fill is typically caused by a small amount of sample creeping up the side of the capillary. Although we have found that it is typically not necessary for the sample to cover the entire counter electrode to give a valid reading, covering only a very small area as happens with creep fill can give a depressed analyte measurement response. Creep fill can be detected by providing the meter with a threshold current value which the current between the working and counter electrodes must exceed within a preset time after the working electrode is wetted. If a current less than the threshold is measured, an alert condition will be triggered and the user may be prompted to discard the biosensor and try again with a new biosensor.

In a preferred aspect, the biosensor is used for testing for an analyte, notably glucose or cholesterol, in whole blood. Accordingly, another aspect of the present invention provides a biosensor for indicating electrochemically the catalytic activity of an enzyme in the presence of whole blood containing an analyte acted upon by said enzyme, the biosensor comprising:
(a) a first substrate;
(b) a second substrate overlying at least a part of the first substrate;
(c) a working electrode on one of the substrates, the working electrode including a catalytically-active quantity of said enzyme;
(d) a counter electrode on one of the substrates;
(e) a first conductive track connected to said working electrode for making a first electrical connection with a test meter apparatus;
(f) a spacer layer having a channel therein and disposed between the first substrate and the second substrate, the spacer layer channel co-operating with adjacent surfaces to define a capillary flow path which extends from an edge of at least one of said substrates to said electrodes;
   the electrodes being arranged such that a fluid sample which flows evenly along the capillary flow path from said edge will substantially completely cover the working electrode before the fluid sample makes contact with any part of the counter electrode; and the biosensor further comprising
(g) a second conductive track connected to said working electrode for making a second electrical connection with a test meter apparatus;
wherein the electrical resistance measurable between the first conductive track and the second conductive track will be substantially different when the working electrode is wetted by a sample of whole blood than when the working electrode is not wetted by an applied fluid.

Any working electrode may be used providing that its resistance in the absence of whole blood is substantially different than when it is wetted by blood in the capillary flow path. Suitable working electrodes are described in WO 2004/008130, in the name of the present applicant. We have surprisingly found in practice that wetting such working electrodes with whole blood, or with a control solution, causes an increase in resistance.

Other aspects and benefits of the invention will appear in the following specification, drawings and claims.

### BRIEF DESCRIPTION OF THE DRAWINGS

The invention will now be further described, by way of example, with reference to the following drawings in which:
Figure 1 shows stages in the formation of a biosensor in accordance with a first embodiment of the present invention;
Figures 2 and 3 show part of a biosensor in accordance with alternative embodiments of the invention;
Figures 4 and 5 are schematic circuit diagrams showing other embodiments of the invention;
Figure 6 is a graph showing the shift in resistance values for a biosensor in accordance with an embodiment of the invention when the working electrode is wetted with whole blood;
Figure 7 is graph showing the shift in resistance values for a biosensor in accordance with an embodiment of the invention when the working electrode is wetted with a control solution; and
Figure 8 is a flow chart illustrating a method in accordance with a further aspect of the invention.

### DEFINITIONS

When used herein, the following definitions define the stated term:
"Amperometry" is an electrochemical technique in which electrons are added (reduction) or removed (oxidation) from a substance at an electrode surface. The signal measured is current. The term includes steady-state Amperometry, chronoamperometry, and Cottrell-type measurements.
A "biological fluid" is any body fluid in which the analyte can be measured. Examples include blood, sweat, urine, interstitial fluid, dermal fluid, and tears.
A "biosensor" is a device in which a biological element (eg enzyme, antibody, DNA, RNA, receptors, organelles, cells, tissue etc) is in intimate contact with a transducer (eg an electrode) which transduces a biological or chemical reaction into an electrical signal which can be measured and related to the amount of an analyte in a test sample.
"Blood" includes whole blood and fluid components of whole blood, for example plasma and serum.
"Coulometry" is the determination of charge passed or projected to pass during complete or near-complete electrolysis of the analyte. The determination may be made using measurements of a decaying current and elapsed time during electrolysis of a sample.
A "counter electrode" is one or more electrodes paired with the working electrode, through which passes a current equal in magnitude and opposite in sign to the current passed through the working electrode. The term includes counter electrodes which also function as reference electrodes.
"Electrolysis" is the electrooxidation or electroreduction of a compound either directly at an electrode or via one or more mediators.
A "faradaic current" is a current corresponding to the reduction or oxidation of a chemical substance. The net faradaic current is the algebraic sum of all the faradaic currents flowing through a working electrode.
"Potentiometry" is the measurement of electrical potential under conditions of low or no current flow, which may be used to determine the presence or quantity of analyte in a fluid.
A "reference electrode" is an electrode that has a substantially stable equilibrium electrode potential. It can be used as a reference point against which the potential of other electrodes, notably the working electrode, can be measured. The term includes reference electrodes which also function as counter electrodes, as is the case in the experimental biosensors of the present application.
A "working electrode" is an electrode at which a substance undergoes electrolysis to produce an electrical signal (eg current, potential) which can be correlated to the amount of analyte in a test sample.

### DETAILED DESCRIPTION

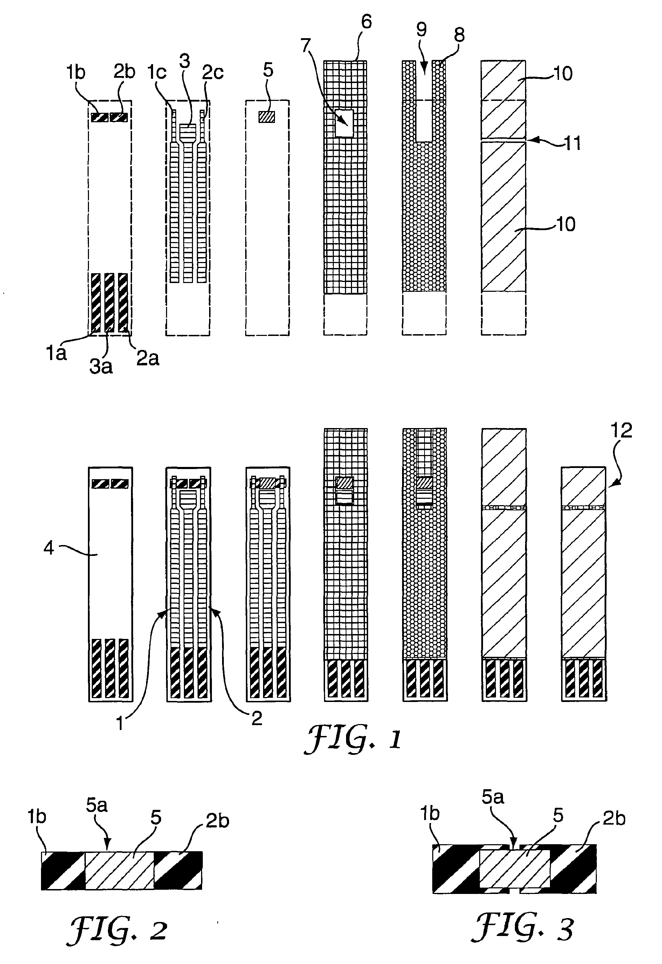

A biosensor 12 in accordance with an embodiment of the invention is made by a series of steps as illustrated in Figure 1. The top row of Figure 1 illustrates a process step, and the bottom row illustrates the sequential build-up of the biosensor 12.

A base substrate 4 is provided, in this example formed from a polyester material (Valox^{™}). Conductive contacts 1 a, 2a, 3a, 1b and 2b are printed onto the base substrate 4 as a conductive carbon paste, product code C80130D1, Gwent Electronic Materials, UK. After printing, the ink of the conductive contacts is dried for 1 minute in a forced air dryer at 130°C. A silver/silver chloride paste is printed as a central print, which will provide the counter electrode 3, and as a first outer print 1 c and a second outer print 2c. The central silver/silver chloride print overlies part of the central conductive contact 3a, connecting it to the counter electrode 3. The first outer print 1 c overlies parts of the conductive carbon contacts 1 a and 1 b to define in combination a first conductive track 1. The second outer print 2c overlies parts of the conductive carbon contacts 2a and 2b to define in combination a second conductive track 2.

A working electrode 5 is formed on the base substrate 4 so that it partially overlies the conductive contacts 1b and 2b of the conductive tracks 1 and 2. The first and second conductive tracks 1, 2 are joined only by the working electrode 5. A dielectric layer 6 is printed, excluding a working area 7 in which the working electrode 5 and counter electrode 3 are located.

The working electrode 5 in this example is formed by printing an ink containing platinised carbon and a resin, drying the printed ink, and applying to the dried ink an aqueous solution containing glucose oxidase, a buffer and trehalose.

### Ink Formulation

| | |
|---|---|
| Metech 8101 resin | 45.32% |
| BSA-Pt/Carbon | 18.67% |
| graphite | 9.77% |
| BCA/cyclohexanone | 23.26% |
| Tween® 20 | 2.98% |

The resin, solvent and flow agent were initially blended together prior to adding the carbon fraction. Initially the formulation was hand mixed followed by several passes through a triple roll mill. This produces a smooth homogeneous thixotropic carbon ink suitable for screen-printing.

Tween 20 is a surfactant supplied by Sigma-Aldrich. Tween is a registered trade mark of ICI Americas Inc. The solvent is a 50% v/v mixture of Butyl Cellosolve Acetate (BCA) and cyclohexanone. The graphite was Timrex KS 15 (particle size < 16 µm), from GS Inorganics, Evesham, Worcs. UK.

### Preparation of Drop Coating Solution

The drop-coating solution comprised:

| | | |
|---|---|---|
| Buffer KH₂PO₄/K₂HPO₄ | 385 mM, pH 8 | Sigma |
| Enzyme Glucose oxidase | 4080 U/mL | Biozyme |
| Stabiliser Trehalose | 1% | Sigma |

The activity of the glucose oxidase is about 270 units per milligram of material (360 units/mg of protein because the enzyme comes in a preparation with other lyophilisation and stabilisation agents).

The drop coat solution was applied to the working electrode using BioDot drop coating apparatus. The volume of drop coating solution used was about 150 nL; this was dried in a forced air drier for 1 minute at 50°C.

A spacer layer 8 is applied over the dielectric layer 6. In the present example, the spacer layer 8 is formed from double-sided adhesive tape of thickness about 90 µm. The tape is Adhesives Research 90118, comprising a 26 µm PET carrier with two 32 µm AS-110 acrylic medical-grade adhesive layers. The spacer 8 has a channel 9 which will determine the capillary flow path of the biosensor. A second substrate, or lid, 10 is adhered to the spacer 8. The lid 10 comprises a 100 µm PET tape (Adhesive Research 90119) coated with about 12.5 µm of a hydrophilic heat-seal adhesive 'HY10'. The lid 10 is provided with a narrow vent 11 to permit the exit of air from the capillary flow path. The vent 11 need not extend right across the lid 10 but could comprise a hole or short slot in fluid communication with the capillary flow path. Finally, the second substrate 10 is guillotined to produce the biosensor 12. Alternatively the spacer 8 could, of course, be initially adhered to the second substrate 10 and then adhered to the first substrate. A benefit of this arrangement is that the second substrate 10 may be cut to provide the vent 11 while both parts of the second substrate 10 are held in the correct positions by the spacer 8.

The biosensor 12 has a capillary flow path defined by the channel 9 in the spacer 8, the inner surface of the lid 10, and the first substrate 2 (largely covered by the dielectric layer 6). The flow path extends from the parallel short edges of each of the substrates 4, 10 to the counter and working electrodes 3, 5. The inner surface of the lid 10 is treated to be hydrophilic to facilitate wetting by blood. With glucose oxidase as the enzyme, the biosensor is used to measure blood glucose. It will be understood that by using other enzymes, other analytes may be measured instead of glucose.

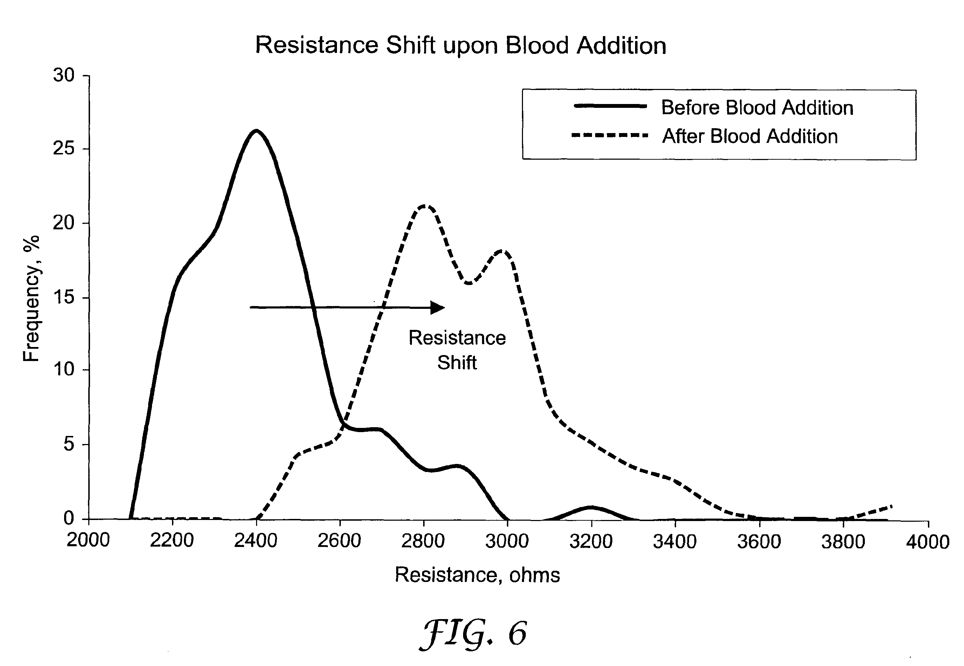

Experimental results for resistance measurements between tracks 1 and 2 (ie across the working electrode 5) of the biosensor 12 are shown in Figure 6, both before (solid line) and after (broken line) wetting of the working electrode 5 by whole blood *via* the capillary flow path. The average resistance before blood application was 2400 ohms, which increased by about 20% to 2900 ohms when blood was applied. The resistance range before blood application was from 2100 to 3200 ohms, and the range after blood application was 2400 to 3800 ohms. For all measurements made there was always an increase in resistance. The average increase was 19.5%, the minimum was 11 % and the maximum was 27%. The measurements were performed on blood with haematocrits from 30-55%; no haematocrit dependence was found.

Resistance measurement results (kΩ) for whole blood at a range of glucose concentrations and with different working electrodes are given in Tables 1-6. In each table the resistance of the working electrode before and after wetting with whole blood is given, and the % change is calculated as the difference divided by the dry resistance. The results in Tables 1-3 are for a design as shown in Figure 1, in which the working electrode 5 has a greater area than the underlying carbon tracks 1b, 2b. The results in Tables 4-6 are for a design as shown in Figure 3, in which the underlying carbon tracks 1b, 2b have a greater area than the working electrode 5. In each case, the resistance increased by at least 10%. The average values for each set of measurements are summarised in Table 7, and the combined average values for both designs are summarised in Table 8. As can be seen from the results, there is substantially no glucose dependency in the resistance results. In these examples, the arrangement of Figure 3 gave a marginally greater average resistance shift, but either arrangement is suitable for distinguishing the wetted working electrode from the dry working electrode. Thus, for the exemplified system, a measured increase in resistance across the working electrode 5 may be used as a diagnostic indicator that the working electrode has been wetted by a sample of blood. In the present example, an increase of about 10% or greater denotes substantially complete wetting of the working electrode by whole blood. It will be understood that the change in resistance need not necessarily be an increase; a consistent decrease in resistance, such as may be provided by alternative working electrode formulations, would be equally useful.

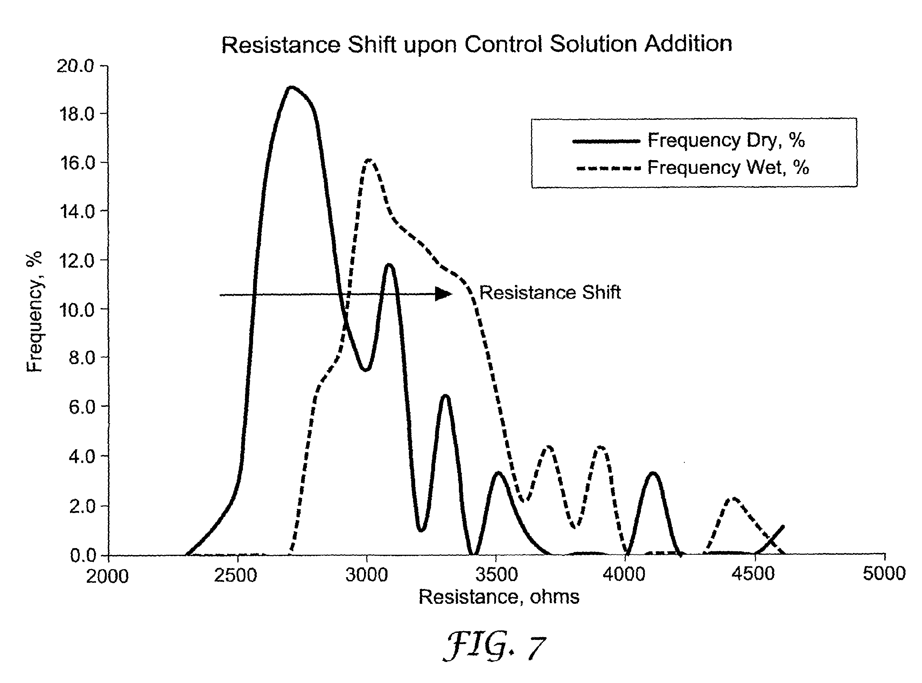

Results for control solutions used to calibrate meters are shown in Figure 7. The control solutions were P/N 410005 Advance Micro-draw L1 and P/N 410006 Advance Micro-draw L1/L2. The L1 solution has a glucose concentration of 80 mg/dl and the L2 solution has a concentration of 250 mg/dl. We found no significant difference in resistance shifts for the two solutions. In each measurement, the resistance of the working electrode increased when wetted by the control solution. The minimum increase was 5% and the maximum was 18%, with an average increase of about 12%. Thus an increase in measured resistance across the working electrode of 5% or more may be used as an indicator that the working electrode has been adequately wetted by control solution or by whole blood.

**Table 1 - FIG. 1, 65 mg/dl**

| Before | After | Difference | % change |
|---|---|---|---|
| 2.508 | 3.105 | 0.60 | 23.8 |
| 2.732 | 3.189 | 0.46 | 16.7 |
| 2.967 | 3.409 | 0.44 | 14.9 |
| 2.669 | 3.158 | 0.49 | 18.3 |
| 3.892 | 4.4 | 0.51 | 13.1 |
| 2.548 | 2.957 | 0.41 | 16.1 |
| 2.833 | 3.221 | 0.39 | 13.7 |
| 2.631 | 3.009 | 0.38 | 14.4 |
| 2.665 | 3.063 | 0.40 | 14.9 |
| 2.537 | 2.979 | 0.44 | 17.4 |
| 2.633 | 3.038 | 0.41 | 15.4 |
| 2.55 | 2.935 | 0.39 | 15.1 |
| 3.011 | 3.414 | 0.40 | 13.4 |
| 2.445 | 2.779 | 0.33 | 13.7 |
| 2.935 | 3.319 | 0.38 | 13.1 |
| 2.763 | 3.152 | 0.39 | 14.1 |

**Table2 ― FIG. 1, 220 mg/dl**

| Before | After | Difference | % change |
|---|---|---|---|
| 2.507 | 2.968 | 0.46 | 18.4 |
| 2.58 | 3.033 | 0.45 | 17.6 |
| 3.032 | 3.52 | 0.49 | 16.1 |
| | | | |
| 2.978 | 3.397 | 0.42 | 14.1 |
| 2.425 | 2.806 | 0.38 | 15.7 |
| 2.663 | 3.002 | 0.34 | 12.7 |
| 2.722 | 3.08 | 0.36 | 13.2 |
| 2.688 | 3.044 | 0.36 | 13.2 |
| 2.805 | 3.18 | 0.38 | 13.4 |
| 3.106 | 3.476 | 0.37 | 11.9 |
| 2.884 | 3.277 | 0.39 | 13.6 |
| 3.074 | 3.475 | 0.40 | 13.0 |
| 2.621 | 3.016 | 0.40 | 15.1 |
| 2.579 | 2.958 | 0.38 | 14.7 |
| 2.644 | 3.009 | 0.37 | 13.8 |

**Table 3 - FIG. 1, 598 mg/dl**

| Before | After | Difference | % change |
|---|---|---|---|
| 4.85 | 5.83 | 0.98 | 20.2 |
| 2.945 | 3.426 | 0.48 | 16.3 |
| 3.296 | 3.797 | 0.50 | 15.2 |
| 2.295 | 2.756 | 0.46 | 20.1 |
| 2.813 | 3.294 | 0.48 | 17.1 |
| 2.644 | 3.062 | 0.42 | 15.8 |
| 2.63 | 2.988 | 0.36 | 13.6 |
| 2.603 | 3.03 | 0.43 | 16.4 |
| 2.341 | 2.751 | 0.41 | 17.5 |
| 2.677 | 3.066 | 0.39 | 14.5 |
| 2.809 | 3.174 | 0.37 | 13.0 |
| 3.337 | 3.714 | 0.38 | 11.3 |
| 2.567 | 2.965 | 0.40 | 15.5 |
| 2.849 | 3.207 | 0.36 | 12.6 |
| 3.391 | 3.779 | 0.39 | 11.4 |
| 3.173 | 3.499 | 0.33 | 10.3 |
| 2.495 | 2.879 | 0.38 | 15.4 |

**Table 4 - FIG. 3, 65 mg/dl**

| Before | After | Difference | % change |
|---|---|---|---|
| 2.474 | 3.02 | 0.55 | 22.1 |
| 2.509 | 3.081 | 0.57 | 22.8 |
| 2.435 | 2.977 | 0.54 | 22.3 |
| 2.516 | 2.992 | 0.48 | 18.9 |
| 2.534 | 3.039 | 0.51 | 19.9 |
| 2.373 | 2.915 | 0.54 | 22.8 |
| 4.47 | 4.97 | 0.50 | 11.2 |
| 2.994 | 3.529 | 0.54 | 17.9 |
| 2.714 | 3.123 | 0.41 | 15.1 |
| 3.381 | 3.855 | 0.47 | 14.0 |
| 2.771 | 3.209 | 0.44 | 15.8 |
| 2.418 | 2.957 | 0.54 | 22.3 |
| 2.391 | 2.83 | 0.44 | 18.4 |
| 2.576 | 2.987 | 0.41 | 16.0 |
| 2.4998 | 2.972 | 0.47 | 18.9 |

**Table 5 - FIG. 3, 220 mg/dl**

| Before | After | Difference | % change |
|---|---|---|---|
| 2.584 | 3.217 | 0.63 | 24.5 |
| 3.08 | 3.67 | 0.59 | 19.2 |
| 2.618 | 3.088 | 0.47 | 18.0 |
| 2.502 | 3.016 | 0.51 | 20.5 |
| 2.469 | 2.948 | 0.48 | 19.4 |
| 2.461 | 2.957 | 0.50 | 20.2 |
| 2.743 | 3.212 | 0.47 | 17.1 |
| 4.58 | 5.06 | 0.48 | 10.5 |
| 2.782 | 3.293 | 0.51 | 18.4 |
| 2.394 | 2.844 | 0.45 | 18.8 |
| 2.385 | 2.777 | 0.39 | 16.4 |
| 2.677 | 3.118 | 0.44 | 16.5 |
| 2.518 | 2.919 | 0.40 | 15.9 |
| 3.709 | 4.2 | 0.49 | 13.2 |
| 2.757 | 3.177 | 0.42 | 15.2 |
| 2.667 | 3.085 | 0.42 | 15.7 |

**Table 6 - FIG. 3, 598 mg/dl**

| Before | After | Difference | % change |
|---|---|---|---|
| 2.478 | 3.009 | 0.53 | 21.4 |
| 2.356 | 2.865 | 0.51 | 21.6 |
| 2.551 | 3.046 | 0.50 | 19.4 |
| 2.528 | 3.104 | 0.58 | 22.8 |
| 2.995 | 3.514 | 0.52 | 17.3 |
| 2.403 | 2.878 | 0.48 | 19.8 |
| 2.858 | 3.148 | 0.29 | 10.1 |
| 2.331 | 2.762 | 0.43 | 18.5 |
| 2.665 | 3.107 | 0.44 | 16.6 |
| 2.592 | 2.985 | 0.39 | 15.2 |
| 2.681 | 3.053 | 0.37 | 13.9 |
| 2.464 | 2.918 | 0.45 | 18.4 |
| 2.444 | 2.829 | 0.39 | 15.8 |
| 5.51 | 5.91 | 0.40 | 7.3 |
| 2.551 | 3.006 | 0.46 | 17.8 |
| 2.628 | 3.103 | 0.48 | 18.1 |
| 2.494 | 2.915 | 0.42 | 16.9 |

**Table 7**

| Design | Blood Glucose, mg/dl | Mean Dry Resistance, kΩ | Mean Wet Resistance, kΩ | Mean Change, kΩ | % Change |
|---|---|---|---|---|---|
| FIG. 1 | 65 | 2.77 | 3.20 | 0.43 | 15 |
| FIG. 1 | 220 | 2.75 | 3.15 | 0.40 | 14 |
| FIG. 1 | 598 | 2.95 | 3.40 | 0.44 | 15 |
| FIG.3 | 65 | 2.74 | 3.23 | 0.49 | 19 |
| FIG. 3 | 220 | 2.81 | 3.29 | 0.48 | 17 |
| FIG.3 | 598 | 2.76 | 3.21 | 0.45 | 17 |

**Table 8**

| Blood Glucose, mg/dl | Mean Dry Resistance, kΩ | Mean Wet Resistance, kΩ | Mean Change, kΩ | % Change |
|---|---|---|---|---|
| 65 | 2.75 | 3.21 | 0.46 | 17.02 |
| 220 | 2.78 | 3.22 | 0.44 | 15.95 |
| 598 | 2.86 | 3.30 | 0.44 | 16.09 |

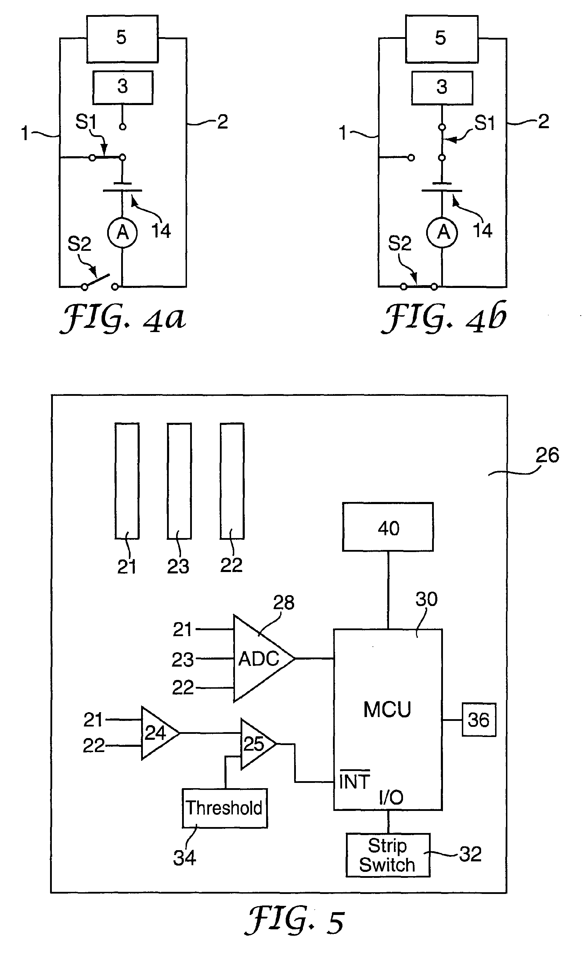

The schematic electric circuit shown in Figure 4 illustrates two modes of operation for measuring currents through parts of the exemplified biosensor 12. In Figure 4a, a two-way first switch S1 is in a first configuration and a second switch S2 is open. A voltage source 14 applies a voltage across the working electrode 5 via tracks 1 and 2. A current is measured (in this example by means of an ammeter). When a change in current is measured indicative of wetting of the working electrode 5 by a fluid sample, the circuit is re-configured by moving the first switch S1 to its other configuration and closing the second switch S2. This arrangement polarises the working electrode 5 relative to the reference/counter electrode 3 so that the measured current will be proportional to the analyte (glucose) concentration in the applied fluid sample when the fluid sample has adequately bridged the two electrodes 5, 3.

When the biosensor 12 is to be used to take a blood glucose reading, the user inserts it into a meter so that the conductive pads 1 a, 2a, 3a are brought into electrical contact with corresponding contacts on the meter. Making these contacts completes a circuit and alerts the meter to expect a reading to be taken.

The meter applies a potential difference across the first conductive track 1 and the second conductive track 3 and measures the resulting current through the working electrode 5, which is related to the resistance of the circuit by Ohm's law. In the absence of a sample of a biological fluid, the working electrode 5 has a lower resistance and the measured current is above a pre-set threshold. Alternatively, the resistance may be calculated by measuring a change in potential from a constant current. A change in impedance could also be measured; measuring AC impedance may have advantages as the impedance may be tuned to give a frequency that has greater sensitivity than DC resistance. In another embodiment, a ratiometric change may be measured for the resistance and/or impedance instead of a simple threshold value. This approach allows for any batch-to-batch differences, and differences in factors such as temperature, sample matrix and other parameters. Various means for measuring resistance and/or impedance will be well known to those skilled in the art of electronics.

In a prior art device, the meter has a first stored threshold value which is used to diagnose used, wet, or otherwise contaminated test strips. This stored threshold has a single defined value. The meter detects any current flow above a threshold when a test strip is added to the meter (before analyte measurement) so there needs to be enough fluid already on the strip to bridge the working and counter electrodes. In the present invention, contamination from blood or other fluid on the working electrode is detected by measuring impedance or resistance between the first and second conductive tracks, even if the fluid does not bridge the working and counter electrodes.

In the present example, a user may take a glucose reading by lancing an alternative site such as his or her upper arm to produce a small drop of blood on the skin, and touching the free short edge of the biosensor 12 to the skin where the blood is located. The blood is drawn rapidly through the channel 9 of the capillary flow path while displaced air exits through the vent 11. When blood wets the working electrode 5 the conductivity of the working electrode 5 substantially decreases (ie, the resistance substantially increases), resulting in a reduced current through the first and second conductive tracks 1, 2 via the working electrode 5. When this current is below a pre-set threshold, indicating that the working electrode 5 is adequately covered by the sample, the meter initiates a timing sequence and switches its electronics to a two electrode potentiostat in which the working electrode 5 is polarised relative to the counter electrode 3. If within a predetermined time no circuit is made between the working electrode 5 and the counter electrode 3 then an error condition will be reported, indicative of an insufficient volume of blood having been added to the biosensor 12.

In a preferred embodiment, the meter is provided with a threshold value against which the current between the working electrode 5 and counter electrode 3 is measured. If no current is measured within the specified time, this indicates that there is insufficient sample such that none has reached the counter electrode. The user may be prompted to discard the biosensor and try again. Alternatively, the user may be prompted or permitted to add a second blood sample to augment the existing sample. This 'double-dosing' does not reduce accuracy because it does not produce an additional non-faradaic charging peak when contact is first made between the working and counter electrodes. If a current is measured which is greater than zero but less than the threshold value, this indicates 'creep fill' in which a thin bead of sample has connected the electrodes, but in a quantity insufficient to provide an accurate reading. In this event the meter will trigger an error condition and the user will be prompted to discard the biosensor and try again with a fresh biosensor.

Thus, the invention provides a biosensor that will allow the detection of blood or other fluids entering the capillary flow path by only covering , partially covering or reaching the working electrode and not reaching, or not reaching sufficient of, the counter electrode.

Other embodiments of the invention are illustrated in Figures 2 and 3. While the working electrode 5 of the biosensor of Figure 1 extends further upstream in the capillary flow path than the contact pads 1 b, 2b, the working electrode 5 could alternatively be printed so that its most upstream surface 5a is level with the most upstream surfaces of the contact pads 1b, 2b, as shown in Figure 2. This arrangement ensures that any change in resistance between the first and second conductive tracks correlates to a wetting of the working electrode 5 by the sample. However, it would also be possible for the footprint of the contact pads 1 b, 2b to extend beyond the footprint of the working electrode 5 in the upstream direction, as shown in Figure 3, so that the change in resistance measurement could relate to the conductive path through the added fluid between the two exposed front shoulders of the conductive pads 1b, 2b as well as the change in resistance of the working electrode 5.

Referring now to Figure 5, an embodiment of a meter 26 for use in the present invention is illustrated. The meter 26 includes a first contact 21, a second contact 22 and a third contact 23, each of which can have a voltage applied to it. When the biosensor 12 is to be used to take a blood glucose reading, the user inserts it into the meter 26 so that the conductive pads 1a, 2a, 3a are brought into electrical contact with, respectively, corresponding contacts 21, 22, 23. Insertion of the biosensor activates a strip-detection switch 32, sending a signal to a micro-control unit (MCU) 30 and alerts the meter to expect a reading to be taken. A timer 36 is started by the MCU 30. An analog to digital converter 28 provides the MCU 30 with digital voltage values for each of the three contacts 21, 22, 23. A first comparator 24 compares the potential difference between contacts 21 and 22 (ie the potential difference across the working electrode 5) at constant current and compares this difference to a threshold level. If the potential difference exceeds a threshold (indicating that the working electrode 5 has been wetted by a blood sample), the working electrode 5 is polarised relative to the counter electrode 3 and the voltage value is input to a second comparator 25, which compares the voltage with a threshold value 34. A voltage above threshold generates a HIGH output (corresponding to no current between the working electrode 5 and the counter electrode 3. The HIGH output does not trigger the MCU 30 to calculate a glucose concentration reading. If the output remains HIGH until the timer 36 records a time-out, the MCU 30 causes a display screen 40 to display a time out message, optionally with a notification that the user may apply a second sample of blood (double dosing). If the voltage compared by the second comparator 25 is below threshold (indicating that a fluid sample is connecting the working electrode 5 and the counter electrode 3) the second comparator 25 outputs a LOW signal to the MCU 10, which calculates a current value from the inputs from the ADC 28. If the calculated value is below a pre-set threshold (indicative of creep-fill), the MCU 30 causes the display 40 to display a creep-fill error and prompt the user to discard the biosensor 12 and start again with a fresh biosensor. If the calculated current value exceeds the threshold, the MCU 30 calculates the glucose concentration of the sample using the current value together with any batch calibration information with which it has been programmed, and outputs the concentration value *via* the display 40.

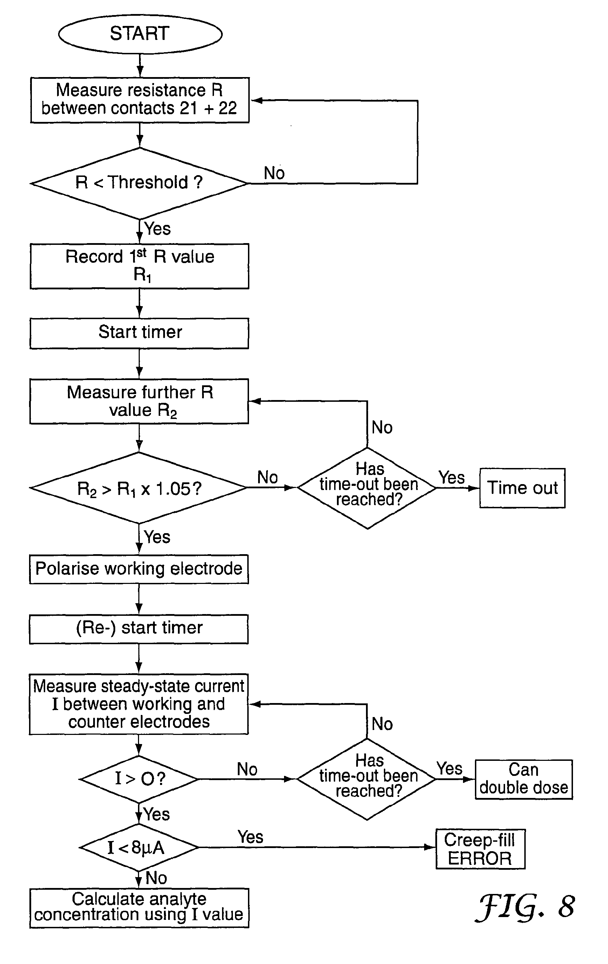

As an alternative to using a conventional strip switch 32 to determine when a biosensor (test strip) has been inserted in the meter, the invention can instead measure an electrical property between the contacts 21 and 22 (corresponding to tracks 1 and 2 on the biosensor 12). An example process is shown in the flow diagram of Figure 8. From an initial start point in which no biosensor is in the meter, electrical resistance R is measured between the contacts 21 and 22. If R is greater than or equal to a threshold value (for example 10 kΩ), no biosensor is inserted. If R is less than the threshold value, a strip is determined to be inserted. The initial resistance value R₁ is recorded and a timer is started. The meter continues to measure resistance values (R₂) and determines whether R₂ is more than 5% higher than R₁. If not, resistance measurement continues until time out (for example after two minutes from insertion of the biosensor). In the present example, if R₂ is more than 5% higher than R₁, this indicates that the working electrode 5 has been wetted by the sample (for example whole blood or control solution).

When the meter has determined that the working electrode has been wetted, it polarises the working electrode 5 relative to the counter electrode 3 and re-starts the timer. In this example, the working electrode is polarised to about 350 mV. The current between the working electrode 5 and counter electrode 3 is monitored. If no current flows within a pre-set time (for example 30 seconds) after wetting of the working electrode, the meter signals a time out condition, but may prompt the user to double dose by adding a further blood sample to the biosensor.

If the measured current is greater than zero amperes but less than a threshold value (in the example less than 8 µA), the meter signals a creep-fill error condition and prompts the user to discard the biosensor and start again with a fresh biosensor. The user is not given the option of double dosing because this would cause inaccuracy in the measured glucose value.

If the measured current is greater than or equal to the threshold value, the meter determines that an adequate sample has been added and that no creep-fill condition exists. The current value is used to calculate a glucose concentration which is displayed to the user.

It is appreciated that certain features of the invention, which are for clarity described in the context of separate embodiments, may also be provided in combination in a single embodiment. Conversely, various features of the invention which are, for the sake of brevity, described in the context of a single embodiment, may also be provided separately or in any suitable subcombination.

While the present invention has been described with reference to specific embodiments, it should be understood that modifications and variations of the invention may be constructed without departing from the scope of the invention defined in the following claims.

## Claims

1. A biosensor which includes a working electrode and a counter electrode, wherein at least a part of the working electrode is disposed between and in electrical connection with two separate conductive tracks.

2. A biosensor according to claim 1, wherein the working electrode and the counter electrode are located in a capillary flow path which extends inwardly from an edge of the biosensor.

3. A biosensor according to claim 2, wherein the electrodes are arranged such that a fluid sample which flows along the capillary flow path from said edge will substantially completely cover the working electrode before the fluid sample makes contact with any part of the counter electrode.

4. A biosensor according to any preceding claim, wherein the electrical resistance measurable between said conductive tracks will be substantially different when the working electrode is substantially covered with whole blood than said electrical resistance in the absence of whole blood.

5. A biosensor according to claim 1, for indicating electrochemically the catalytic activity of an enzyme in the presence of whole blood containing an analyte acted upon by said enzyme, the biosensor further comprising:
(a) a first substrate;
(b) a second substrate overlying at least a part of the first substrate;
(c) a spacer layer having a channel therein and disposed between the first substrate and the second substrate, the spacer layer channel co-operating with adjacent surfaces to define a capillary flow path which extends from an edge of at least one of said substrates to said electrodes;
the electrodes being arranged such that a fluid sample which flows evenly along the capillary flow path from said edge will substantially completely cover the working electrode before the fluid sample makes contact with any part of the counter electrode;
wherein the working electrode is located on one of the substrates and includes a catalytically-active quantity of the enzyme;
the counter electrode is located on one of the substrates;
and wherein the electrical resistance measurable between the conductive tracks will be substantially different when the working electrode is wetted by a sample of whole blood than when the working electrode is not wetted by an applied fluid.

6. A biosensor according to claim 5 or claim 6, wherein the electrical resistance measurable between the conductive tracks will be substantially higher when the working electrode is wetted by a sample of whole blood than when the working electrode is not wetted by an applied fluid.

7. A biosensor according to any preceding claim, wherein each of said two separate conductive tracks includes a contact pad; the working electrode being disposed over both contact pads.

8. A biosensor according to claim 7, wherein said contact pads are formed from a conductive carbon material.

9. A biosensor according to claim 5, wherein the working electrode is disposed over an end of each of the two conductive tracks.

10. A biosensor according to claim 9, wherein the working electrode is located such that a fluid sample which flows evenly along the capillary flow path from said edge will make contact with part of the working electrode before it makes contact with both of the conductive tracks.

11. A biosensor according to claim 9, wherein the working electrode is located such that a fluid sample which flows evenly along the capillary flow path from said edge will make contact with both of the conductive tracks before it makes contact with any part of the working electrode.

12. A biosensor according to claim 9, wherein the working electrode is located such that a fluid sample which flows evenly along the capillary flow path from said edge will make contact with the working electrode at substantially the same time that it makes contact with both of the conductive tracks.

13. A test meter for measuring analyte concentration in a biological fluid sample applied to a biosensor; the meter having a first electrical contact , a second electrical contact, and a third electrical contact, each for making an electrical connection with a corresponding contact pad on a biosensor;
means for measuring an electrical property between said first and second contacts;
means for applying a potential across the first electrical contact and the third electrical contact depending on whether the measured electrical property exceeds or does not exceed a first threshold value within a first pre-set time;
means for measuring an electric current value through said first and third contacts when said potential is applied;
means for converting said electric current value into an analyte concentration value; and
means for displaying said analyte concentration value.

14. A test meter according to claim 13, wherein the measured electrical property is an electric current value and wherein said potential will be applied between the first electrical contact and the third electrical contact if the measured electrical property exceeds said first threshold value within said first pre-set time.

15. A test meter according to claim 14, further comprising means for triggering a first error condition if said electric current value between the first and third contacts is greater than zero but less than a second threshold value after a second pre-set time.

16. A test meter according to claim 15, further comprising means for triggering a second error condition if said electric current value between the first and third contacts is zero after said second pre-set time.

17. A test meter according to claim 13, wherein the measured electrical property is a resistance value or an impedance value and wherein said potential will be applied between the first electrical contact and the third electrical contact if the measured electrical property is above said first threshold value within said first pre-set time.

18. A method for determining whether a fluid sample applied to a biosensor as claimed in any of claims 1-8 has sufficiently wetted the working electrode; the method comprising:
a) measuring a first value of an electrical property of the working electrode;
b) comparing said first value with a first value range;
c) if said first value is within said first value range, determining that the working electrode has been sufficiently wetted by the fluid sample;
e) if said first value is not within said first value range, determining that the working electrode has not been sufficiently wetted.

19. A method according to claim 18, wherein the first value is a current through the working electrode via the conductive tracks; the method further comprising the steps of:
a) measuring a second value of a current through the working electrode and the counter electrode a pre-determined time after measuring the first value;
b) determining a ratio value of a ratio of said second value to said first value;
c) triggering an error condition if said ratio value is less than a preset ratio threshold value.

20. A method according to claim 18, wherein said electrical property comprises impedance, resistance, current or potential difference.

21. A method according to any of claims 18-20, further comprising removing any applied potential between the conductive tracks if it is determined that the working electrode has been sufficiently wetted.

22. A method according to claim 21, further comprising applying a potential between the working electrode and the counter electrode, measuring a current value through the working electrode and the counter electrode and comparing that current value with a threshold value.

23. A method according to claim 22, further comprising triggering a first error condition if said current value is greater than zero but less than the threshold value after a pre-set time.

24. A method according to claim 23, further comprising triggering a second error condition if said current value is zero after said pre-set time.

25. A method according to claim 22, further comprising converting said current value into an analyte concentration value and displaying said analyte concentration value if said current value equals or exceeds said threshold value.

26. A method according to claim 18, comprising:
a) applying a potential across the working electrode *via* the conductive tracks;
b) measuring a first value of resistance of the working electrode within a first preset time after applying said potential;
c) comparing said first value with a first threshold value;
d) if said first value is above said first threshold value, determining that the working electrode has been sufficiently wetted by the fluid sample;
e) if said first value is not above said first threshold value, determining that the working electrode has not been sufficiently wetted.

27. A method according to claim 18, comprising:
a) applying a potential across the working electrode *via* the conductive tracks;
b) measuring a first current value through the working electrode within a first preset time after applying said potential;
c) comparing said first current value with a first threshold value;
d) if said first current value does not exceed said first threshold value, determining that the working electrode has been sufficiently wetted by the fluid sample;
e) if said first current value exceeds said first threshold value, determining that the working electrode has not been sufficiently wetted.

28. A method according to claim 18, comprising:
a) causing a current to flow through the working electrode via the conductive tracks;
b) measuring a first value of potential difference across the working electrode within a first pre-set time after initially causing said current to flow;
c) comparing said first value with a first threshold value;
d) if said first value is above said first threshold value, determining that the working electrode has been sufficiently wetted by the fluid sample;
e) if said first value is not above said first threshold value, determining that the working electrode has not been sufficiently wetted.

29. A method according to claim 18, wherein said electrical property comprises a ratiometric value derived from the value for resistance or impedance between the conductive tracks before and after the application of a fluid sample to the biosensor.

30. A method of manufacturing a biosensor, comprising: providing a first substrate and a second substrate, providing two separate conductive tracks on one of said substrates and forming a working electrode on that substrate so that at least a part of the working electrode is disposed between and in electrical connection with both conductive tracks, and providing a counter electrode on one of the substrates.

31. A method according to claim 30, wherein each of said two separate conductive tracks includes a contact pad; the working electrode being disposed over both contact pads.

32. A method according to claim 31, wherein said contact pads are formed from a conductive carbon material.
